# EUROPEAN PATENT SPECIFICATION

(11) **EP 4 226 875 B1**
(45) Date of publication and mention of the grant of the patent: **23.04.2025**
(21) Application number: 22155808.3
(22) Date of filing: 09.02.2022
(51) Int. Cl.: A61B 17/225, A61B 17/00

(54) **SHOCK WAVE DEVICE HAVING A SOURCE SELF ALIGNING WITH AN X-RAY DEVICE**
STOSSWELLENVORRICHTUNG MIT EINER QUELLE, DIE SICH SELBST AUF EINE RÖNTGENVORRICHTUNG AUSRICHTET
DISPOSITIF À ONDES DE CHOC DOTÉ D'UNE SOURCE À ALIGNEMENT AUTOMATIQUE AVEC UN DISPOSITIF À RAYONS X

(43) Date of publication of application: 16.08.2023
(73) Proprietor: Storz Medical AG, 8274 Tägerwilen (CH)
(72) Inventor: Storz, Rafael, 8280 Kreuzlingen (CH); Marlinghaus, Ernst, 8598 Bottighofen (CH); Kühl, Arvid, 8274 Tägerwilen (CH)
(74) Representative: Lohr, Jöstingmeier & Partner

(56) References cited:
- EP-A1- 2 628 456
- EP-A1- 3 875 048
- WO-A2-2008/048708
- US-A1- 2010 080 349
- US-A1- 2016 114 194
- US-A1- 2019 350 685

## Description

### Field of the invention

The invention relates to an extracorporeal shock wave or ultrasound therapy system, e.g., a lithotripsy system or Lithotripter for non-invasive treatment of stones, like e.g., kidney stones, urinary stones, gallstones, or other calculi within a mammal's body using acoustic pulses, or other applications of an extracorporeal shock wave or ultrasound therapy system.

### Description of the related art

In a lithotripsy system, X-ray images are taken from a patient. These images are used for locating a stone and for aligning a therapy source like a shock wave or ultrasound therapy source. Such an X-ray image shows a projection into a plane orthogonal to the X-ray beam, herein referred ax X-Y plane. An information about the height, herein the Z-axis is not available. When the X-ray source is rotated about a predetermined angle alpha about a rotation axis parallel to the X-Y plane, all points at the rotation axis remain at their position.

In view of this, the lithotripsy system is calibrated such that the focal area of the therapy source is at the rotation axis. This is done by taking at least two X-ray images under different angles alpha1 and alpha2 and comparing the positions of an object, e.g., stone or calibration object in the X-ray images. If the position differs, the object is not at the rotation axis. Normally, the X-ray system is moved to bring the object into the rotation axis.

EP 150 674 1 A1 discloses a lithotripsy system where the X-ray system can be mechanically coupled to the shockwave source for alignment. The axis of the x-ray system should be parallel to the axis of the shockwave source, further the focal area of the shockwave source should be on the axis of the x-ray system. This allows for proper triangulation and targeting. Mechanically coupling is a straightforward approach which requires a complex mechanical system and prevents separating the X-ray device from the shockwave device.

EP 2 628 456 A1 discloses a shock wave therapy device with dynamic target tracking. US 2010/080349 A1 discloses a breast biopsy device. EP 3 875 048 A1 discloses a shockwave therapy system with 3D control. US 2019/0350685 A1 discloses an electromagnetic navigation system.

### Summary

The problem to be solved by the invention is to provide an ultrasound and/or shock wave device, including an ultrasound and/or shockwave source and an X-ray system wherein the ultrasound and/or shockwave source is automatically aligned with the X-ray system without mechanically coupling between the ultra-sound and/or shockwave source without mechanically coupling. The ultrasound and/or shock wave device should be comparatively robust and simple to use. Solutions of the problem according to the invention are defined in the independent claims. The dependent claims relate to further improvements of the invention.

In an embodiment, a shock wave and/or ultrasound device, which may be a lithotripter includes an ultrasound and/or shockwave source together with an X-ray system including an X-ray source and a detector. It may further include a patient table. The ultrasound and/or shockwave source is suspended on a hexapod, such that it can be displaced in three degrees of freedom and tilted or rotated about two or three degrees of freedom. Therefore, no complex X-ray system mechanism and complex X-ray adjustment procedures are required. The X-ray system may only be aligned coarsely. The treatment source may then be adjusted by the hexapod drive such that the axes of the X-ray system and the treatment source are the same. As the hexapod allows a free orientation of the source in space, it can basically adjust the treatment source to any orientation of the X-ray source.

The X-Ray system, the ultrasound and/or shockwave source are oriented in a cartesian coordinate system as follows: a y-axis may be a longitudinal axis through the center of the table. An x-axis may be orthogonal to the y-axis and in the plane of the table surface. A z-axis is orthogonal to the plane of the table surface and therefore orthogonal to the x-axis and the y-axis and in a direction upward from the table. There may also be a first rotation around the x-axis, a second rotation and a third rotation around the z-axis. A positive rotation may be a clockwise rotation in a direction of a positive axis.

The orientation of the ultrasound and/or shockwave source is defined by an axis extending from the center of the source to the center of the focal area. The orientation of the X-ray system is defined by an axis at the path of the X-ray beam between the X-ray source and the detector.

The shock wave or ultrasound therapy system may include a system controller which may control at least the X-ray system and the hexapod drive of the ultra-sound and/or shockwave source. The system controller may control the X-ray system for taking at least one X-ray image which may show a known object, which may be a calibration object or at least a part of the ultrasound and/or shockwave source. The known object, may be a calibration phantom or focal phantom and may include a first X-ray absorbing object and a second X-ray absorbing object arranged distant from each other symmetrically to or at the axis of the ultrasound and/or shockwave source. Both X-ray absorbing objects may have different sizes and a symmetrical structure. They may be rings of different diameters. The known object may also be part of the structure of the shock wave and/or ultrasound therapy system, which is visible in an X-ray image.

The system controller may be configured to control the X-ray system to take a first image showing a first X-ray absorbing object and a second X-ray absorbing object.

The system controller may be configured to process the first image, to detect the first and second X-ray absorbing objects and to estimate the displacement of axes between a center axis of the ultra-sound and/or shockwave source and a center axis of the X-ray system. This may be done based on calculating the centers of and/or the distance between the X-ray absorbing objects.

The system controller may further be configured to control the hexapod drive for moving of the ultrasound and/or shockwave source to compensate for the displacement of axes.

The system controller may further be configured to control the X-ray system to take a second image showing a first X-ray absorbing object and a second X-ray absorbing object.

The system controller may be configured to process the second image, to detect the first and second X-ray absorbing objects and to estimate the displacement of axes between a center axis of the ultra-sound and/or shockwave source and a center axis of the X-ray system. The system controller may further be configured to control the hexapod drive for further adjustment if the displacement of axes is above a limit value. This may be repeated until the displacement of axes is below or equal to the limit value or a maximum count of iterations have been reached.

The system controller may be configured to take at least a further image after changing alignment of the ultrasound and/or shockwave source to verify the alignment. It may further be configured to repeat the steps above until an alignment within predetermined limits is reached or an operator is satisfied, or a maximum number of cycles is reached.

The ultrasound and/or shockwave source may be of any type suitable for generating shock waves. It may include a shock wave generator and/or transducer, which may include at least one of a coil, a spark gap or a Piezo transducer. The shock wave generator/transducer may be partially enclosed by a reflector. Depending on the type of transducer, the reflector may have a parabolic or half-elliptic shape. In case of a piezo transducer, the transducer may itself have a spherical shape, such that a reflector may not be needed. The ultrasound and/or shockwave source may have a focal volume which is distant from the ultrasound and/or shockwave source and normally around a center axis of the ultrasound and/or shockwave source. The focal volume may be defined as a volume, where the maximum shock wave intensity is maintained with a deviation of maximal -3 dB or -6 dB. If the focal volume is defined with a 6 dB deviation, the pressure at the limit of the zone is half of the maximum pressure inside the zone. The focal volume may have an elliptical shape with a length in an axial direction (defined by the center axis) of the ultrasound and/or shockwave source axis of 10 to 15 cm and a diameter between 5 and 15 mm. The focal volume normally is spaced from the shock wave generator and/or transducer.

The hexapod drive bearing the ultrasound and/or shockwave source is also known as a hexapod platform. Such a hexapod platform also is called a Stewart platform. Basically, it is a type of parallel manipulator or parallel robot that has six linear actuators, which may be hydraulic or pneumatic jacks or electric linear actuators. Examples of electric linear actors are motors coupled to a belt or a spindle to perform a linear movement. These linear actuators are connected in pairs to three mounting positions at a base, crossing over to three mounting positions at the ultrasound and/or shockwave source. Each connection of a linear actuator to either the base or the ultrasound and/or shockwave source may include a universal joint, also called a cardan joint or a ball joint. By variation of the length of the linear actuators, the ultrasound and/or shockwave source can be moved in six degrees of freedom with respect to the base. There are three degrees of translation and three degrees of rotation. Although the use of six linear actuators is preferred, a lower number of actuators may be used, e.g., like in a delta robot with three actuators.

The hexapod drive allows to adjust the position of an ultrasound and/or shockwave source relative to a patient's body and/or the X-ray system. Positioning of the ultrasound and/or shockwave source may be done automatically or by manual control. An automatic control may allow quick adjustment and it may also allow to store and retrieve preconfigured settings.

Such a hexapod drive is a very robust and mechanical stiff support or suspension of the ultrasound and/or shockwave source. Therefore, it can withstand high forces from the patient body, the weight of the ultrasound and/or shockwave source and dynamic loads which occur when shockwave pulses are generated. Further, a hexapod drive can be moved quickly. Therefore, a stable positioning of the ultrasound and/or shockwave source is achieved providing the additional ability to quickly correct deviations and movements by the patient.

A hexapod drive may allow movement in all 6 degrees of freedom. This allows for a precise adjustment of the position of the focal volume of the ultrasound and/or shockwave source and/or the path of the ultrasound and/or shockwave though the body of the patient.

The patient table may have a basically planar surface defining a longitudinal axis. It is configured for accommodating a patient. The ultrasound and/or shockwave source may be mounted below the patient table. In general, a shockwave source may be mounted in alternative ways, e.g., on a stand or support.

The base may be standing on a floor directly or by a stand or it may be attached to a floor. The base may also hold the table.

A method of aligning an ultrasound and/or shockwave source with an X-ray system includes the steps of:
a) taking a first image with the X-ray system at a first position of the X-ray system relative to the ultrasound and/or shockwave source, the image showing a first X-ray absorbing object and a second X-ray absorbing object arranged distant from each other at the axis of the ultrasound and/or shockwave source,
b) using the first image, calculating the centers of and/or the distance between the first X-ray absorbing object and the second X-ray absorbing object, and estimating the displacement of axes between a center axis of the ultrasound and/or shockwave source and a center axis of the X-ray system by a system controller,
c) moving of the ultrasound and/or shockwave source by means of a hexapod drive to compensate for the displacement of axes,
d) taking a second image with the X-ray system at a second position of the X-ray system, the image showing a first X-ray absorbing object and a second X-ray absorbing object,
e) using the second image, calculating the centers of and/or the distance between the first X-ray absorbing object and the second X-ray absorbing object, and estimating the displacement of axes between a center axis of the ultrasound and/or shockwave source and a center axis of the X-ray system by the system controller.
f) repeating the sequence from step c) if displacement of axes is above a limit value.

If the axes are not aligned and the displacement of axes is above a limit value, the steps c), d) and e) may be repeated multiple times until the displacement of axes is below a limit value.

The X-ray system, the ultrasound and/or shockwave source are oriented in a cartesian coordinate system as defined above.

### Description of Drawings

In the following the invention will be described by way of example, without limitation of the general inventive concept, on examples of embodiment with reference to the drawings.
Figure 1 shows an embodiment of a known lithotripsy system.
Figure 2 shows a perspective view of an ultrasound and/or shockwave source mounted to a hexapod drive.
Figure 3 shows an embodiment of a therapy system.
Figure 4 shows a flow diagram of a method.
Figure 5 shows a sketch of a focal phantom.
Figures 6 to 8 show X-ray images made with the procedure steps.
Figure 9 shows an embodiment in two tilted positions.

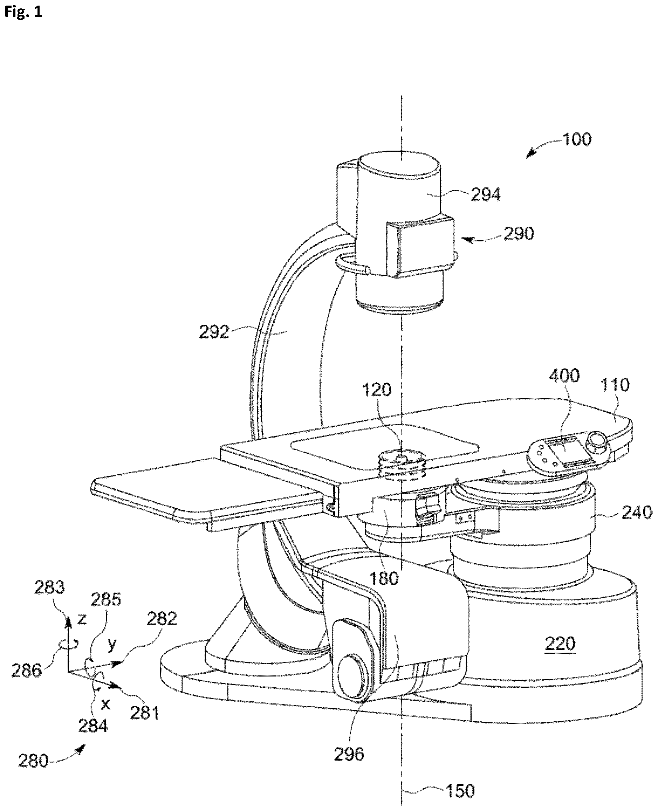

In Figure 1, a first embodiment of a lithotripsy system, known from EP 3 875 048 A1, is shown. The invention works with any ultrasound and/or shockwave treatment system.

An extra-corporeal ultrasound and/or shockwave lithotripsy system for non-invasive treatment of stones 100 comprises a patient table 110, an ultra-sound and/or shockwave source 120. The ultrasound and/or shockwave source 120 is mounted to a hexapod drive 180. The hexapod drive 180 may further be held by a stand 220. The hexapod drive 180 allows fine positioning of the ultrasound and/or shockwave source 120 in multiple axes relative to the patient table 110 and therefore relative to the patient (not shown in this figure). The ultrasound and/or shockwave source 120 has a focal volume which moves together with the source. In an embodiment the distance of the focal volume to the source may be modified.

The patient table 110 is based on a stand 220 which may stand on a floor. The table 110 may be held by a positioning device 240 to move the table relative to the ultrasound and/or shockwave source 120 together with the hexapod drive 180. A movement of the patient table may be coarse positioning which may be limited to a displacement in 3 axes.

The table 110 may have a flat surface for accommodating a patient who is not shown here.

An X-ray device 290, acting as targeting device, may be provided. It may have an X-ray tube 296 opposing an X-ray detector 294, both mounted tiltable at a C-arm 292. There is a common center axis 150 on which the X-ray device and the ultra-sound and/or shockwave source are aligned.

To describe the relative movement of the table 110, the X-ray device 290 and the ultrasound and/or shockwave source 120, a cartesian coordinate system 280 may be used. There is a y-axis 282, which may be a longitudinal axis through the center of the table. Furthermore, there is an x-axis 281 orthogonal to the y-axis and in the plane of the table surface. A z-axis 283 is orthogonal to the plane of the table surface and therefore orthogonal to the x-axis and the y-axis. There may also be a first rotation 284 around the x-axis, a second rotation 285 and a third rotation 286 around the z-axis. A positive rotation may be a clockwise rotation in a view along a positive axis.

A hexapod drive 180 may allow movement in all these 6 degrees of freedom. This allows for a precise adjustment of the position of the focal volume of the ultra-sound and/or shockwave source 120. The patient table may be positioned for a slow and coarse adjustment in 3 degrees of translation for larger distances, but normally no rotation, whereas the hexapod drive provides a comparatively quick adjustment in 3 degrees of translation and 3 degrees of rotation. The movement distances of the table may be larger than the movement distances of the hexapod drive. The X-ray device may at least be tiltable to perform a second rotation 285 around an axis parallel to the y-axis 282. It may also be tiltable around an axis parallel to the Y axis. It may further be translated in a plane defined by the X and Y axis. This means, it may be moved on the floor which is also in the X-Y plane.

For control of the movement of the ultrasound and/or shockwave source 120 relative to the patient table 110, a control panel 400 may be provided.

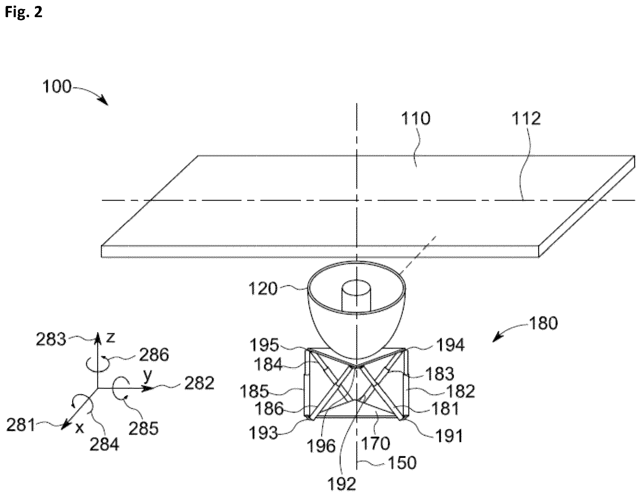

Figure 2 shows a more schematic perspective view of an ultrasound and/or shockwave source 120 mounted to a hexapod drive 180. A shock wave or ultra-sound device, which may be a lithotripter 100 may include a patient table 110 and an ultrasound and/or shockwave source 120. The ultrasound and/or shockwave source 120 may be arranged below the patient table 110, such that a patient (not shown here) may be accommodated on top of the patient table. The patient table may have a longitudinal axis 112. There may be a hole or cutout in the patient table at the position of the ultrasound and/or shockwave source.

The ultrasound and/or shockwave source 120 is supported by and/or suspended on a hexapod drive 180. Basically, it is a type of parallel manipulator or parallel robot that has six linear actuators 181 - 186, which may be hydraulic or pneumatic jacks or electric linear actuators. These linear actuators are connected in pairs to three mounting positions 191, 192, 193 at a base 170, crossing over to three mounting positions 194, 195, 196 at the ultrasound and/or shockwave source 120. Each connection of a linear actuator to either the base or the ultra-sound and/or shockwave source may include a universal joint, also called a cardan joint or a ball joint. By variation of the length of the linear actuators, the ultrasound and/or shockwave source can be moved in six degrees of freedom with respect to the base. There are three degrees of translation, parallel to at least one X-axis 281, Y-axis 282, Z-axis 283 and three degrees of tilt or rotation including a first tilt 284 around an axis parallel to X-axis 281, a second tilt 285 around an axis parallel to Y-axis 282, a third tilt 285 around an axis parallel to Z-axis 283.

Further, the ultrasound and/or shockwave source 120 may have a center axis 150.

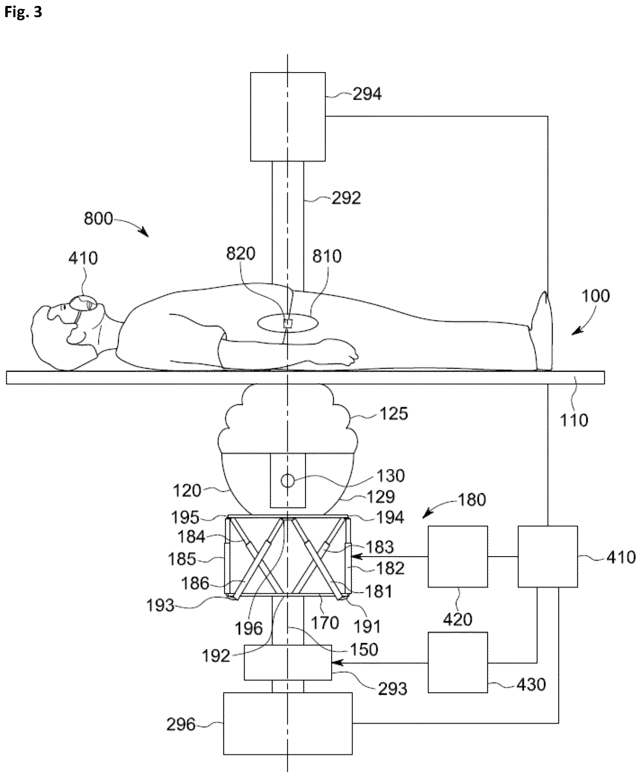

Figure 3 shows an embodiment in a side view. In this figure, a patient 800 is positioned on top of the patient table 110. The patient may have a kidney 810 with a kidney stone 820. An X-ray system includes an X-ray tube 294 shown below the patient and an X-ray detector 294 shown above the patient, both connected and held by a C-arm 292. Here, the X-ray system is aligned with the shock wave generator 130 such that both are on the axis 150. The X-ray system may further include a C-arm drive 293, which may be an electrical motor with a gear.

A system controller controls at least operation of the X-ray system 290, of the C-arm by means a C-arm control 430 and of the hexapod drive 180 by means of a hexapod control 420. The system controller may also control the X-ray tube 296 and may receive images from the X-ray detector 294.

Below the table 110, the ultrasound and/or shockwave source 120 is shown in a sectional view. It may have a shock wave generator 130 which may be a coil as shown herein and which is at least partially enclosed by a reflector 129. Further, the center axis 150 is marked as a dashed line. Normally, the interior of the reflector and the space between the source and the patient is filled with a liquid like water or another shockwave conducting medium. To contain the water within the volume, a coupling cushion 125 may be provided.

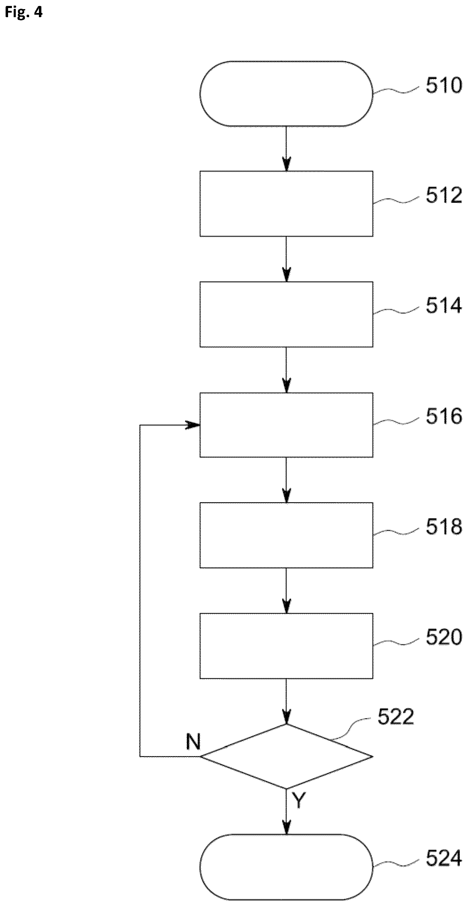

Figure 4 shows a flow diagram of a method of aligning an ultrasound and/or shockwave source 120 with an X-ray system. The method utilizes a focal phantom including a first X-ray absorbing object 127 and a second X-ray absorbing object 128 arranged distant from each other at the axis of the ultrasound and/or shockwave source 120. The method including the steps of:
510: START
512: taking a first image with the X-ray system at a first position of the X-ray system 290 relative to the ultrasound and/or shockwave source 120, the image showing a first X-ray absorbing object 127 and a second X-ray absorbing object 128 arranged distant from each other at the axis of the ultrasound and/or shockwave source 120,
514: processing of the first image, calculating the centers of and/or the distance between the first X-ray absorbing object 127 and the second X-ray absorbing object 128, and estimating the displacement of axes between a center axis of the ultrasound and/or shockwave source 120 and a center axis the axis of the X-ray system 290 by a system controller,
516: moving of the ultrasound and/or shockwave source 120 by means of a hexapod drive 180 to compensate for the displacement of axes and/or relative rotations,
518: taking a second image, the image showing the first X-ray absorbing object 127 and the second X-ray absorbing object 128,
520: processing of the second image, calculating the centers of and/or the distance between the first X-ray absorbing object 127 and the second X-ray absorbing object 128, and estimating the displacement between the axis of the ultra-sound and/or shockwave source 120 and the X-ray system 290 by a system controller,
522: checking whether the axes are aligned and there is no displacement or a displacement below a threshold between the images;
If not aligned (N), go to 516, if yes (Y) go to 524
524: END

Steps 516 to 522 may be repeated multiple times until the offset between the images may be within a predefined limit or further iterations do not reduce the offset.

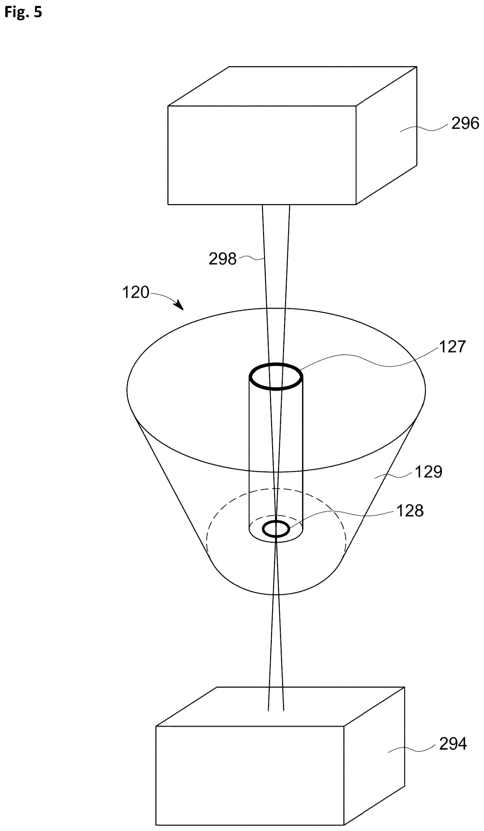

Figure 5 shows a sketch of a focal phantom. An ultrasound and/or shockwave source 120 may have a first X-ray absorbing object 127 and a second X-ray absorbing object 128 distant in z-direction from each other. Both X-ray absorbing objects may be arranged symmetrical to the center axis of the source. Both X-ray absorbing objects may have different sizes and a symmetrical structure. They may be rings of different diameters. The first X-ray absorbing object 127 and a second X-ray absorbing object 128 may be removable from the ultrasound and/or shockwave source 120.

In this embodiment, a shock wave source having a centered cylindrical coil 122 within a reflector 129 is shown. The first X-ray absorbing object 127 may be at one end of the coil, where the second X-ray absorbing object 128 may be at the other side thereof. An X-ray beam 298 from X-ray tube 294 passes through the source 120 and produces images of the Y-ray absorbing objects at the detector. The axis of the ultrasound and/or shockwave source 120 may be moved by the hexapod drive 180 until the images of both X-ray absorbing objects are centered. In this case, the source 120 and the X-ray system 290 are on the same axis.

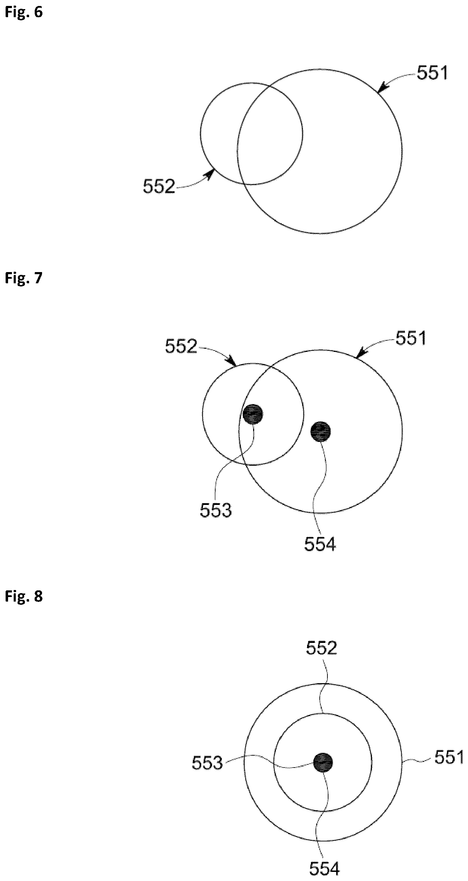

Figures 6 to 8 show X-ray images made with the procedure steps as explained in Fig. 4 with a focal phantom of Fig. 5. Here, Fig. 6 shows an X-ray image as made in step 512. It may show an X-ray image 551 of first X-ray absorbing object 127 and an X-ray image 552 of second X-ray absorbing object 128. If the axis of the X-ray system 290 is not aligned with the axis of the ultrasound and/or shockwave source 120, the objects are not centered. In step 514 the center 554 of X-ray image of first X-ray absorbing object and the center 553 of X-ray image of second X-ray absorbing object are determined. Fig. 8 shows the image taken in step 518 with calculated centers from step 520, which overlap, as in this example the axis may be aligned. If the axes are still not aligned, then the centers would not be at the same position. In this case another iteration may be made.

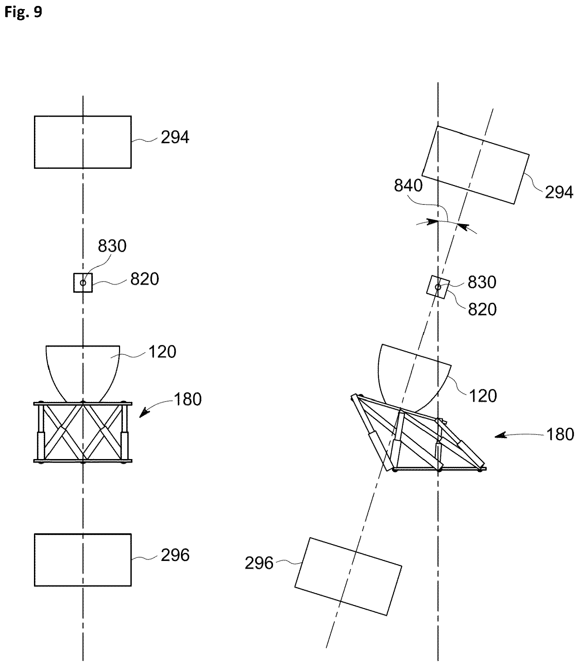

Figure 9 shows an embodiment in two tilted positions. At the left side, a shock wave and/or ultrasound therapy system is shown in vertical position. At the right side, the same system is shown in position tilted at an angle 840 around an axis 830 at the center of stone 820. Here the tilt axis is aligned with an axis of a stone. The image would look the same if the tilt axis is aligned with an axis of a known object or a phantom.

### List of reference numerals

- 100: shock wave and/or ultrasound therapy system
- 110: patient table
- 112: longitudinal axis
- 120: ultrasound and/or shockwave source
- 122: coil
- 125: coupling cushion
- 127: first X-ray absorbing object
- 128: second X-ray absorbing object
- 129: reflector
- 130: shock wave generator
- 150: center axis
- 170: base
- 180: hexapod drive
- 181-186: linear actuators
- 191-193: mounting positions at base
- 194-196: mounting positions at ultrasound and/or shockwave source
- 220: stand
- 240: positioning device
- 280: cartesian coordinate system
- 281: x-axis
- 282: y-axis
- 283: z-axis
- 284: tilt around the x-axis
- 285: tilt around the y-axis
- 286: tilt around the z-axis
- 290: X-ray system
- 292: C-arm
- 293: C-arm drive
- 294: X-ray detector
- 296: X-ray tube
- 298: X-ray beam
- 400: control panel
- 410: system controller
- 420: hexapod control
- 430: C-arm control
- 510 - 524: Method steps
- 551: X-ray image of first X-ray absorbing object
- 552: X-ray image of second X-ray absorbing object
- 553: center of X-ray image of second X-ray absorbing object
- 554: center of X-ray image of first X-ray absorbing object
- 800: patient
- 810: kidney
- 820: kidney stone
- 830: tilt axis
- 840: tilt angle

## Claims

1. A shock wave and/or ultrasound therapy system (100) comprising an ultra-sound and/or shockwave source (120) and an X-ray system (290),
**characterized in that**
the ultrasound and/or shockwave source (120) is suspended on a hexapod drive (180), and a system controller (410) is provided and configured to generate control signals for the hexapod drive (180) to align the ultrasound and/or shockwave source (120) with the X-ray system (290).

2. The shock wave and/or ultrasound therapy system (100) according to claim 1,
**characterized in that**
the system controller (410) is configured to generate control signals to align the ultrasound and/or shockwave source (120) with the X-ray system (290) based on the displacement of a known object between two images taken at different tilt angles of the X-ray system.

3. The shock wave and/or ultrasound therapy system (100) according to any of the preceding claims,
**characterized in that**
the hexapod drive (180) includes six linear actuators (181-186) which are attached in pairs to three positions (191-193) at the base (170), crossing over to three mounting positions (194-196) at the ultrasound and/or shockwave source (120).

4. The shock wave and/or ultrasound therapy system (100) according to any of the preceding claims,
**characterized in that**
each connection of a linear actuator (181-186) to either the base (170) or the ultrasound and/or shockwave source (120) includes a universal joint or a ball joint.

5. The shock wave and/or ultrasound therapy system (100) according to any of the preceding claims,
**characterized in that**
the linear actuators (181-186) include at least one of hydraulic or pneumatic jacks or electric linear actuators.

6. The shock wave and/or ultrasound therapy system (100) according to any of the preceding claims,
**characterized in that**
the hexapod drive (180) is configured to provide movement with at least five degrees of freedom including displacement along three orthogonal axes (281, 282, 283) and at least two degrees of tilt.

7. The shock wave and/or ultrasound therapy system (100) according to any of the preceding claims,
**characterized in that**
the system controller (410) is configured to align:
an axis of the ultrasound and/or shockwave source (120) defined by extending from the center of the source to the center of the focal area with an axis of the X-ray system (290) defined by the path of the X-ray beam between the X-ray source and the detector.

8. The shock wave and/or ultrasound therapy system (100) according to any of the preceding claims,
**characterized in that**
the shock wave and/or ultrasound therapy system (100) includes a focal phantom which further includes a first X-ray absorbing object (127) and a second X-ray absorbing object (128) distant from each other in direction of a center axis of the ultrasound and/or shockwave source (120) and arranged symmetrically to the center axis of the ultrasound and/or shockwave source (120).

9. The shock wave and/or ultrasound therapy system (100) according to the previous claim,
**characterized in that**
the system controller (410) is configured to control the X-ray system (290) for taking at least one X-ray image, and for calculating the centers of and/or the distance between a first X-ray absorbing object (127) and the second X-ray absorbing object (128), and estimating the displacement of axes between a center axis of the ultrasound and/or shockwave source (120) and a center axis of the X-ray system (290) by a system controller.

10. The shock wave and/or ultrasound therapy system (100) according to the previous claim,
**characterized in that**
the system controller (410) is configured to calculate a displacement of the ultrasound and/or shockwave source (120) required to align with the X-ray system (290) based on the displacement of the center axes.

11. The shock wave and/or ultrasound therapy system (100) according to any of the preceding claims,
**characterized in that**
the shock wave and/or ultrasound therapy system (100) is arranged in a cartesian coordinate system with a y-axis (282) being a longitudinal axis through the center of a patient table (110), an x-axis (281) being orthogonal to the y-axis and in the plane of a surface of the patient table (110), a z-axis (283) being orthogonal to the plane of the surface of the patient table (110), and therefore orthogonal to the x-axis and the y-axis and in a direction upward from the table.

12. The shock wave and/or ultrasound therapy system (100) according to any of the preceding claims,
**characterized in that**
the ultrasound and/or shockwave source (120) is configured to generate shock waves propagating in a direction to a patient table (110) above the ultrasound and/or shockwave source (120) and having a focal volume (350) above the patient table (110).

13. The shock wave and/or ultrasound therapy system (100) according to claim 8,
**characterized in that**
the focal phantom comprises X-ray absorbing objects having different sizes and a symmetrical structure, which may be rings of different diameters.

14. A method of aligning an ultrasound and/or shockwave source with an X-ray system includes the steps of:
a) taking a first image with the X-ray system (290) at a first position of the X-ray system (290) relative to the ultrasound and/or shockwave source (120), the image showing a first X-ray absorbing object (127) and a second X-ray absorbing object (128) arranged distant from each other at the axis of the ultrasound and/or shockwave source (120),
b) using the first image, calculating the centers of and/or the distance between the first X-ray absorbing object (127) and the second X-ray absorbing object (128), and estimating the displacement of axes between a center axis of the ultrasound and/or shockwave source (120) and a center axis of the X-ray system (290) by a system controller (410),
c) moving of the ultrasound and/or shockwave source (120) by means of a hexapod drive (180) to compensate for the displacement of axes,
d) taking a second image with the X-ray system (290) at a second position of the X-ray system (290), the image showing a first X-ray absorbing object (127) and a second X-ray absorbing object (128),
e) using the second image, calculating the centers of and/or the distance between the first X-ray absorbing object (127) and the second X-ray absorbing object (128), and estimating the displacement of axes between a center axis of the ultrasound and/or shockwave source (120) and a center axis of the X-ray system (290) by a system controller (410),
f) repeating the sequence from step c) if displacement of axes is above a limit value.

## Patentansprüche

1. Ein Stoßwellen- und/oder Ultraschalltherapiesystem (100), umfassend eine Ultraschall- und/oder Stoßwellenquelle (120) und ein Röntgensystem (290),
**dadurch gekennzeichnet, dass**
die Ultraschall- und/oder Stoßwellenquelle (120) an einem Hexapod-Antrieb (180) aufgehängt ist, und
eine Systemsteuerung (410) bereitgestellt und konfiguriert ist, um Steuerungssignale für den Hexapod-Antrieb (180) zu erzeugen, um die Ultraschall- und/oder Stoßwellenquelle (120) mit dem Röntgensystem (290) auszurichten.

2. Das Stoßwellen- und/oder Ultraschalltherapiesystem (100) nach Anspruch 1,
**dadurch gekennzeichnet, dass**
die Systemsteuerung (410) konfiguriert ist, um Steuersignale zum Ausrichten der Ultraschall- und/oder Stoßwellenquelle (120) mit dem Röntgensystem (290) basierend auf der Verschiebung eines bekannten Objekts zwischen zwei Bildern, die bei unter unterschiedlichen Neigungswinkeln des Röntgensystems aufgenommen wurden, zu erzeugen.

3. Das Stoßwellen- und/oder Ultraschalltherapiesystem (100) nach einem der vorhergehenden Ansprüche,
**dadurch gekennzeichnet, dass**
der Hexapod-Antrieb (180) sechs Linearaktuatoren (181-186) umfasst, welche paarweise an drei Positionen (191-193) an der Basis (170) befestigt sind und über drei Befestigungspositionen (194-196) an der Ultraschall- und/oder Stoßwellenquelle (120) verlaufen.

4. Das Stoßwellen- und/oder Ultraschalltherapiesystem (100) nach einem der vorhergehenden Ansprüche,
**dadurch gekennzeichnet, dass**
jede Verbindung eines Linearaktuators (181-186) mit entweder der Basis (170) oder der Ultraschall- und/oder Stoßwellenquelle (120) ein Universalgelenk oder ein Kugelgelenk umfasst.

5. Das Stoßwellen- und/oder Ultraschalltherapiesystem (100) nach einem der vorhergehenden Ansprüche,
**dadurch gekennzeichnet, dass**
die Linearaktuatoren (181-186) mindestens einen hydraulischen oder pneumatischen Zylinder oder elektrische Linearaktuatoren umfassen.

6. Das Stoßwellen- und/oder Ultraschalltherapiesystem (100) nach einem der vorhergehenden Ansprüche
**dadurch gekennzeichnet, dass**
der Hexapod-Antrieb (180) so konfiguriert ist, dass er eine Bewegung mit mindestens fünf Freiheitsgraden bereitstellt, einschließlich einer Verschiebung entlang drei orthogonaler Achsen (281, 282, 283) und mindestens zwei Neigungsgraden.

7. Das Stoßwellen- und/oder Ultraschalltherapiesystem (100) nach einem der vorhergehenden Ansprüche
**dadurch gekennzeichnet, dass**
die Systemsteuerung (410) konfiguriert ist, um auszurichten:
eine Achse der Ultraschall- und/oder Stoßwellenquelle (120), welche durch eine Erstreckung von der Mitte der Quelle zur Mitte des Fokusbereichs definiert ist, mit einer Achse des Röntgensystems (290), die durch den Weg des Röntgenstrahls zwischen der Röntgenquelle und dem Detektor definiert ist.

8. Das Stoßwellen- und/oder Ultraschalltherapiesystem (100) nach einem der vorhergehenden Ansprüche,
**dadurch gekennzeichnet, dass**
das Stoßwellen- und/oder Ultraschalltherapiesystem (100) ein Fokalphantom umfasst, welches weiterhin ein erstes Röntgenstrahlen absorbierendes Objekt (127) und ein zweites Röntgenstrahlen absorbierendes Objekt (128), umfasst, welche in Richtung einer Mittelachse der Ultraschall- und/oder Stoßwellenquelle (120) voneinander beabstandet und symmetrisch zur Mittelachse der Ultraschall- und/oder Stoßwellenquelle (120) angeordnet sind.

9. Das Stoßwellen- und/oder Ultraschalltherapiesystem (100) nach dem vorhergehenden Anspruch,
**dadurch gekennzeichnet, dass**
die Systemsteuerung (410) konfiguriert ist, um das Röntgensystem (290) zum Aufnehmen mindestens eines Röntgenbild zu steuern, und zum Berechnen der Mittelpunkte und/oder des Abstands zwischen einem ersten Röntgenstrahlen absorbierenden Objekt (127) und dem zweiten Röntgenstrahlen absorbierenden Objekt (128), und zum Schätzen der Achsenverschiebung zwischen einer Mittelachse der Ultraschall- und/oder Stoßwellenquelle (120) und einer Mittelachse des Röntgensystems (290) durch eine Systemsteuerung.

10. Das Stoßwellen- und/oder Ultraschalltherapiesystem (100) nach dem vorhergehenden Anspruch,
**dadurch gekennzeichnet, dass**
die Systemsteuerung (410) konfiguriert ist, um eine Verschiebung der Ultraschall- und/oder Stoßwellenquelle (120), die zur Ausrichtung mit dem Röntgensystem (290) erforderlich ist, basierend auf der Verschiebung der Mittelachsen zu berechnen.

11. Das Stoßwellen- und/oder Ultraschalltherapiesystem (100) nach einem der vorhergehenden Ansprüche,
**dadurch gekennzeichnet, dass**
das Stoßwellen- und/oder Ultraschalltherapiesystem (100) in einem kartesischen Koordinatensystem angeordnet ist, wobei eine y-Achse (282) eine Längsachse durch die Mitte eines Patiententisches (110) ist, eine x-Achse (281) orthogonal zur y-Achse und in der Ebene einer Oberfläche des Patiententisches (110) ist, eine z-Achse (283) orthogonal zur Ebene der Oberfläche des Patiententisches (110) und daher orthogonal zur x-Achse und zur y-Achse und in einer Richtung vom Tisch nach oben ist.

12. Das Stoßwellen- und/oder Ultraschalltherapiesystem (100) nach einem der vorhergehenden Ansprüche,
**dadurch gekennzeichnet, dass**
die Ultraschall- und/oder Stoßwellenquelle (120) konfiguriert ist, um Stoßwellen zu erzeugen, die sich in Richtung eines Patiententisches (110) über der Ultraschall- und/oder Stoßwellenquelle (120) ausbreiten und über dem Patiententisch (110) ein Fokusvolumen (350) aufweisen.

13. Das Stoßwellen- und/oder Ultraschalltherapiesystem (100) nach Anspruch 8,
**dadurch gekennzeichnet, dass**
das Fokalphantom Röntgenstrahlen absorbierende Objekte mit unterschiedlichen Größen und einer symmetrischen Struktur, welche Ringe unterschiedlichen Durchmessers sein können, umfasst.

14. Ein Verfahren zum Ausrichten einer Ultraschall- und/oder Stoßwellenquelle mit einem Röntgensystem umfasst die folgenden Schritte:
a) Aufnehmen eines ersten Bildes mit dem Röntgensystem (290) an einer ersten Position des Röntgensystems (290) relativ zur Ultraschall- und/oder Stoßwellenquelle (120), wobei das Bild ein erstes Röntgenstrahlen absorbierendes Objekt (127) und ein zweites Röntgenstrahlen absorbierendes Objekt (128) zeigt, die auf der Achse der Ultraschall- und/oder Stoßwellenquelle (120) voneinander beabstandet angeordnet sind,
b) Berechnen der Mittelpunkte und/oder des Abstands zwischen dem ersten Röntgenstrahlen absorbierenden Objekt (127) und dem zweiten Röntgenstrahlen absorbierenden Objekt (128) anhand des ersten Bildes, und Schätzen der Achsenverschiebung zwischen einer Mittelachse der Ultraschall- und/oder Stoßwellenquelle (120) und einer Mittelachse des Röntgensystems (290) durch eine Systemsteuerung (410),
c) Bewegen der Ultraschall- und/oder Stoßwellenquelle (120) mittels eines Hexapod-Antriebs (180), um Achsenverschiebungen zu kompensieren,
d) Aufnehmen eines zweiten Bildes mit dem Röntgensystem (290) an einer zweiten Position des Röntgensystems (290), wobei das Bild ein erstes Röntgenstrahlen absorbierendes Objekt (127) und ein zweites Röntgenstrahlen absorbierendes Objekt (128) zeigt,
e) Berechnen der Mittelpunkte und/oder des Abstands zwischen dem ersten Röntgenstrahlen absorbierenden Objekt (127) und dem zweiten Röntgenstrahlen absorbierenden Objekt (128) anhand des zweiten Bildes, und Schätzen der Achsenverschiebung zwischen einer Mittelachse der Ultraschall- und/oder Stoßwellenquelle (120) und einer Mittelachse des Röntgensystems (290) durch eine Systemsteuerung (410),
f) Wiederholen der Sequenz ab Schritt c), wenn die Achsenverschiebung über einem Grenzwert liegt.

## Revendications

1. Système de thérapie par ondes de choc et/ou par ultrasons (100) comprenant une source d'ultrasons et/ou d'ondes de choc (120) et un système à rayons X (290),
**caractérisé en ce que**
la source d'ultrasons et/ou d'ondes de choc (120) est suspendue à un organe d'entraînement hexapode (180), et un organe de commande de système (410) est prévu et configuré pour générer des signaux de commande destinés à l'organe d'entraînement hexapode (180) afin d'aligner la source d'ultrasons et/ou d'ondes de choc (120) avec le système à rayons X (290).

2. Système de thérapie par ondes de choc et/ou par ultrasons (100) selon la revendication 1,
**caractérisé en ce que**
l'organe de commande de système (410) est configuré pour générer des signaux de commande afin d'aligner la source d'ultrasons et/ou d'ondes de choc (120) avec le système à rayons X (290) en fonction du déplacement d'un objet connu entre deux images prises à différents angles d'inclinaison du système à rayons X.

3. Système de thérapie par ondes de choc et/ou par ultrasons (100) selon l'une quelconque des revendications précédentes,
**caractérisé en ce que**
l'organe d'entraînement hexapode (180) inclut six actionneurs linéaires (181-186) qui sont fixés par paires en trois positions (191-193) au niveau de la base (170), passant à trois positions de montage (194-196) au niveau de la source d'ultrasons et/ou d'ondes de choc (120).

4. Système de thérapie par ondes de choc et/ou par ultrasons (100) selon l'une quelconque des revendications précédentes,
**caractérisé en ce que**
chaque connexion d'un actionneur linéaire (181-186) à la base (170) ou à la source d'ultrasons et/ou d'ondes de choc (120) inclut un joint universel ou un joint à rotule.

5. Système de thérapie par ondes de choc et/ou par ultrasons (100) selon l'une quelconque des revendications précédentes,
**caractérisé en ce que**
les actionneurs linéaires (181-186) incluent au moins l'un parmi des vérins hydrauliques ou pneumatiques ou des actionneurs linéaires électriques.

6. Système de thérapie par ondes de choc et/ou par ultrasons (100) selon l'une quelconque des revendications précédentes,
**caractérisé en ce que**
l'organe d'entraînement hexapode (180) est configuré pour fournir un mouvement avec au moins cinq degrés de liberté, dont un déplacement le long de trois axes orthogonaux (281, 282, 283) et au moins deux degrés d'inclinaison.

7. Système de thérapie par ondes de choc et/ou ultrasons (100) selon l'une quelconque des revendications précédentes,
**caractérisé en ce que**
l'organe de commande de système (410) est configuré pour aligner :
un axe de la source d'ultrasons et/ou d'ondes de choc (120) défini en s'étendant depuis le centre de la source au centre de la zone focale avec un axe du système à rayons X (290) défini par le chemin du faisceau de rayons X entre la source de rayons X et le détecteur.

8. Système de thérapie par ondes de choc et/ou ultrasons (100) selon l'une quelconque des revendications précédentes,
**caractérisé en ce que**
le système de thérapie par ondes de choc et/ou par ultrasons (100) comprend un fantôme focal qui inclut en outre un premier objet absorbant les rayons X (127) et un second objet absorbant les rayons X (128) éloignés l'un de l'autre dans la direction d'un axe central de la source d'ultrasons et/ou d'ondes de choc (120) et agencés symétriquement par rapport à l'axe central de la source d'ultrasons et/ou d'ondes de choc (120).

9. Système de thérapie par ondes de choc et/ou par ultrasons (100) selon la revendication précédente,
**caractérisé en ce que**
l'organe de commande de système (410) est configuré pour commander le système à rayons X (290) pour prendre au moins une image à rayons X, et pour calculer les centres et/ou la distance entre un premier objet absorbant les rayons X (127) et le second objet absorbant les rayons X (128), et estimer le déplacement d'axes entre un axe central de la source d'ultrasons et/ou d'ondes de choc (120) et un axe central du système à rayons X (290) par un organe de commande de système.

10. Système de thérapie par ondes de choc et/ou par ultrasons (100) selon la revendication précédente,
**caractérisé en ce que**
l'organe de commande de système (410) est configuré pour calculer un déplacement de la source d'ultrasons et/ou d'ondes de choc (120) nécessaire pour s'aligner avec le système à rayons X (290) en fonction du déplacement des axes centraux.

11. Système de thérapie par ondes de choc et/ou ultrasons (100) selon l'une quelconque des revendications précédentes,
**caractérisé en ce que**
le système de thérapie par ondes de choc et/ou par ultrasons (100) est agencé dans un système de coordonnées cartésiennes dont un axe y (282) est un axe longitudinal passant par le centre d'une table de patient (110), un axe x (281) qui est orthogonal à l'axe y et dans le plan d'une surface de la table de patient (110), un axe z (283) qui est orthogonal au plan de la surface de la table de patient (110), et donc orthogonal à l'axe x et à l'axe y et dans une direction vers le haut à partir de la table.

12. Système de thérapie par ondes de choc et/ou par ultrasons (100) selon l'une quelconque des revendications précédentes,
**caractérisé en ce que**
la source d'ultrasons et/ou d'ondes de choc (120) est configurée pour générer des ondes de choc se propageant dans une direction vers une table de patient (110) au-dessus de la source d'ultrasons et/ou d'ondes de choc (120) et ayant un volume focal (350) au-dessus de la table de patient (110).

13. Système de thérapie par ondes de choc et/ou par ultrasons (100) selon la revendication 8,
**caractérisé en ce que**
le fantôme focal comprend des objets absorbant les rayons X ayant différentes tailles et une structure symétrique, qui peuvent être des bagues de différents diamètres.

14. Procédé d'alignement d'une source d'ultrasons et/ou d'ondes de choc avec un système à rayons X incluant les étapes de :
a) prise d'une première image avec le système à rayons X (290) à une première position du système à rayons X (290) par rapport à la source d'ultrasons et/ou d'ondes de choc (120), l'image montrant un premier objet absorbant les rayons X (127) et un second objet absorbant les rayons X (128) agencés à distance l'un de l'autre sur l'axe de la source d'ultrasons et/ou d'ondes de choc (120),
b) à l'aide de la première image, calcul des centres de et/ou de la distance entre le premier objet absorbant les rayons X (127) et le second objet absorbant les rayons X (128), et estimation du déplacement d'axes entre un axe central de la source d'ultrasons et/ou d'ondes de choc (120) et un axe central du système à rayons X (290) par un organe de commande de système (410),
c) mise en mouvement de la source d'ultrasons et/ou d'ondes de choc (120) au moyen d'un organe d'entraînement hexapode (180) pour compenser le déplacement d'axes,
d) prise d'une seconde image avec le système à rayons X (290) à une seconde position du système à rayons X (290), l'image montrant un premier objet absorbant les rayons X (127) et un second objet absorbant les rayons X (128),
e) à l'aide de la seconde image, calcul des centres de et/ou de la distance entre le premier objet absorbant les rayons X (127) et le second objet absorbant les rayons X (128), et estimation du déplacement d'axes entre un axe central de la source d'ultrasons et/ou d'ondes de choc (120) et un axe central du système à rayons X (290) par un organe de commande de système (410),
f) répétition de la séquence de l'étape c) si le déplacement d'axes est supérieur à une valeur limite.
